# EUROPEAN PATENT APPLICATION

(11) **EP 4 123 017 A1**
(43) Date of publication of application: **25.01.2023**
(21) Application number: 21771577.0
(22) Date of filing: 17.03.2021
(51) Int. Cl.: C12N 5/0775, C12N 15/63, A61K 35/34, A61P 9/10

(54) **COMPOSITION FOR PREVENTING OR TREATING ISCHEMIC DISEASES, COMPRISING CARDIAC STEM CELLS**

(30) Priority: 17.03.2020 KR 20200032823
(71) Applicant: Hierabio Inc., Seoul 03760 (KR)
(72) Inventor: LEE, Seung Jin, Seoul 07333 (KR); KIM, Heejung, Goyang-si, Gyeonggi-do 10352 (KR); SHIN, Ji Young, Seoul 03902 (KR); JEON, Jeong Hwa, Seoul 03938 (KR); SEO, Chae Won, Seoul 07380 (KR)
(74) Representative: Studio Torta S.p.A.
(86) International application number: PCT/KR2021/003310
(87) International publication number: WO 2021/187895

(57) **Abstract**

Disclosed is a pharmaceutical composition for preventing or treating angiogenesis-dependent diseases, comprising, as an active ingredient, cardiac stem cells having enhanced angiogenic potential or a culture broth thereof.

## Description

### Technical Field

The present invention relates to cardiac stem cells, cardiac stem cells with enhanced angiogenic potential, a culture broth thereof, and a use thereof, and more particularly to cardiac stem cells, or cardiac stem cells introduced with a gene such as HIF-1α, VEGF, PDGF, HGF, or FGF, and a use thereof.

### Background Art

Angiogenesis, as a phenomenon wherein endothelial cells of pre-existing blood vessels degrade the extracellular matrix, migrate, divide, and differentiate to form new capillary vessels, occurs in physiological processes such as growth, reproduction, and wound healing. Also, angiogenesis is related to pathological conditions such as growth of tumors, arthritis, and diabetes. Vasculogenesis requires a series of complex processes such as growth, migration, differentiation, and capillary formation of vascular endothelial cells, and various angiogenesis-promoting factors and angiogenesis-inhibiting factors involved in these processes have been discovered. The angiogenesis-inhibiting factors are activated to counteract the angiogenesis-promoting factors required for angiogenesis. Since angiogenesis inhibitors naturally existing in a body may be used to suppress pathological angiogenesis due to low toxicity, many drugs related thereto are in development.
While excessive formation of blood vessels may be a major cause of disease exacerbation, non-formation of blood vessels may also be a cause of severe diseases. Angiogenesis is an essential phenomenon required for wound healing or tissue regeneration. For example, undeveloped angiogenesis in placenta may result in miscarriage, and necrosis, ulcer, and ischemia caused by undeveloped angiogenesis may cause dysfunction of tissues or organs, even death. In addition, diseases such as atherosclerosis, myocardial infarction, and angina pectoris are caused by unstable supply of blood. Therefore, there is a need to develop therapeutic methods for inducing or promoting angiogenesis to reduce tissue damage caused by a low-oxygen state or a low-nutrient state due to non-formation of blood vessels and to properly regenerate tissue (Korean Patent Laid-open Publication No. 2009-0010662).

In particular, angiogenesis should be accompanied by a wound healing process that is essential for regenerating wounded skin tissue. At an early state of wound healing, an inflammatory response is caused by necrosis of cells and rupture of blood vessels, and then a series of processes of forming biological mediators such as kallikrein, thrombin, and plasmin may follow after the inflammatory response along with a phenomenon of leaking out blood components, activation of platelets, and blood clotting.

Therefore, therapeutic agents capable of efficiently promoting angiogenesis need to be developed and are expected to be applied to treatment of various diseases such as wounds, chronic ulcer, ischemic stroke, myocardial infarction, angina pectoris, and cerebrovascular dementia.

### Disclosure

### Technical Problem

The present invention has been proposed to solve the above-described problems occurring in the prior art, and an object of the present invention is to provide a pharmaceutical composition for preventing or treating angiogenesis-dependent diseases, the composition including, as an active ingredient, cardiac stem cells, cardiac stem cells having enhanced angiogenic potential by introducing a gene such as HIF-1α, VEGF, PDGF, HGF, or FGF, or a culture broth thereof.

However, objects to be accomplished by the present invention are not limited to the above-mentioned object, and other objects not mentioned above will be clearly understood by those skilled in the art from the following description.

### Technical Solution

The present invention provides a pharmaceutical composition for preventing or treating an angiogenesis-dependent disease including, as an active ingredient, cardiac stem cells or a culture broth thereof. The cardiac stem cells may be cardiac stem cells introduced with a gene encoding at least one growth factor such as hypoxia-inducible factor 1-alpha (HIF-1α), vascular endothelial growth factor (VEGF), platelet-derived growth factor (PDGF), hepatocyte growth factor (HGF), and fibroblast growth factor (FGF), *i.e.,* transformed with the gene to overexpress the gene, wherein the cardiac stem cells introduced with the gene are characterized by having enhanced angiogenic potential. The hypoxia-inducible factor 1-alpha may be mutated such that the 392^{nd} to 520^{th} amino acids are removed, the 567^{th} amino acid proline is substituted with threonine, and the 658^{th} amino acid proline is substituted with glutamine in the amino acid sequence.

In a specific embodiment of the present invention, the cardiac stem cells having enhanced angiogenic potential or the culture broth thereof may include various growth factors such as hypoxia-inducible factor 1-alpha, vascular endothelial growth factor, platelet-derived growth factor, hepatocyte growth factor, and fibroblast growth factor.

In another specific embodiment of the present invention, the angiogenesis-dependent disease collectively refers to all diseases whose symptoms may be ameliorated, alleviated, and treated by promoting angiogenesis, preferably wounds, chronic ulcer, peripheral artery disease, critical limb ischemia, diabetic foot ulcer, ischemic stroke, myocardial infarction, angina pectoris, cerebrovascular dementia, or the like, and more preferably myocardial infarction, angina pectoris, or the like, but the disease is not limited as long as the disease is treated by promoting angiogenesis.

In another specific embodiment of the present invention, the pharmaceutical composition may further include hydrogel, and the hydrogel may be prepared preferably using collagen, gelatin, chondroitin, hyaluronic acid, alginic acid, Matrigel^{™}, chitosan, peptide, fibrin, polyglycolic acid (PGA), polylactic acid (PLA), polyethylene glycol (PEG), and polyacrylamide.

In addition, the present invention provides a method for preventing or treating an angiogenesis-dependent disease, the method including administering a composition to an individual, the composition including, as an active ingredient, cardiac stem cells, cardiac stem cells transformed with a gene encoding a least one growth factor selected from hypoxia-inducible factor 1-alpha (HIF-1α), vascular endothelial growth factor (VEGF), platelet-derived growth factor (PDGF), hepatocyte growth factor (HGF), and fibroblast growth factor (FGF), or a culture broth thereof.

In addition, the present invention provides a use of a composition for preventing or treating an angiogenesis-dependent disease, the composition including, as an active ingredient, cardiac stem cells, cardiac stem cells transformed with a gene encoding a least one growth factor selected from hypoxia-inducible factor 1-alpha (HIF-1α), vascular endothelial growth factor (VEGF), platelet-derived growth factor (PDGF), hepatocyte growth factor (HGF), and fibroblast growth factor (FGF), or a culture broth thereof.

In addition, the present invention provides a use of cardiac stem cells, cardiac stem cells transformed with a gene encoding a least one growth factor selected from hypoxia-inducible factor 1-alpha (HIF-1α), vascular endothelial growth factor (VEGF), platelet-derived growth factor (PDGF), hepatocyte growth factor (HGF), and fibroblast growth factor (FGF), or a culture broth thereof, for producing a drug used for an angiogenesis-dependent disease.

### Advantageous Effects

Cardiac stem cells, cardiac stem cells having enhanced angiogenic potential, or a culture broth thereof according to the present invention may be involved in differentiation, survival, proliferation, angiogenesis, and migration of stem cells by activating an angiogenic gene such as VEGF, angiopoietins, FGF, PLGF, PDGF, SDF-1, and SCF and simultaneously activating a gene related to survival and proliferation of cells such as IGF, TGF-α, TGF-β, and NOS2, thereby significantly promoting angiogenesis of cardiac stem cells. Therefore, the cardiac stem cells having enhanced angiogenic potential or the culture broth thereof according to the present invention are expected to be widely applied to all diseases whose symptoms may be ameliorated, alleviated, and treated by promoting angiogenesis, such as wounds, chronic ulcer, ischemic stroke, myocardial infarction, angina pectoris, and cerebrovascular dementia.

### Brief Description of Drawings

FIG. 1 shows results of identifying expression levels of angiogenesis-related genes in cardiac stem cells and bone marrow mesenchymal stem cells by qRT-PCR according to an embodiment of the present invention.
FIG. 2 shows results of identifying effects of hypoxic conditions on expression level of VEGF in cardiac stem cells by ELISA according to an embodiment of the present invention.
FIG. 3 shows results of identifying effects of hypoxic conditions on expression level of angiogenesis-related genes in cardiac stem cells by qRT-PCR according to an embodiment of the present invention.
FIG. 4 shows results of identifying effects of hypoxic conditions on expression level of HIF-1α in cardiac stem cells by western blotting according to an embodiment of the present invention.
FIG. 5 schematically illustrates a vector map for expressing eGFP or HIF-1α gene according to an embodiment of the present invention.
FIG. 6 shows expression levels of HIF-1α gene in cardiac stem cells transformed with a vector for expressing HIF-1α gene by western blotting according to an embodiment of the present invention.
FIG. 7 shows results of identifying effects of HIF-1α gene on viability of cells using a CCK-8 assay according to an embodiment of the present invention.
FIG. 8 shows results of identifying effects of HIF-1α gene on cellular migration using a wound healing scratch assay according to an embodiment of the present invention.
FIG. 9 shows results of identifying effects of HIF-1α gene on tube formation using a tube formation assay according to an embodiment of the present invention.
FIG. 10 shows results of identifying effects of HIF-1α gene on differentiation into vascular endothelial cell according to an embodiment of the present invention.
FIG. 11 shows results of identifying effects of HIF-1α gene on restoration of damaged blood vessels *in vivo* by measuring blood perfusion according to an embodiment of the present invention.
FIG. 12 shows results of identifying effects of HIF-1α gene on inhibiting muscle damage and fibrosis *in vivo* according to an embodiment of the present invention.
FIG. 13 shows expression levels of HIF-1α gene in cardiac stem cells transformed with a vector for expressing constitutively active HIF-1α gene by western blotting according to an embodiment of the present invention.
FIG. 14 shows results of identifying effects of cardiac stem cells transformed with a vector for expressing constitutively active HIF-1α gene on expression of angiogenesis-related genes using qRT-PCR according to an embodiment of the present invention.
FIG. 15 shows results of identifying effects of constitutively active HIF-1α gene on cellular migration using a wound healing scratch assay according to an embodiment of the present invention.
FIG. 16 shows results of identifying effects of constitutively active HIF-1α gene on tube formation using a tube formation assay according to an embodiment of the present invention.
FIG. 17 shows results of identifying effects of constitutively active HIF-1α gene on differentiation into vascular endothelial cells according to an embodiment of the present invention.
FIG. 18 shows results of identifying characteristics of constitutively active HIF-1α gene-introduced cardiac stem cells encapsulated in fibrin gel according to an embodiment of the present invention. A and B show results of viability of cells in fibrin gel, C shows results of identifying degrees of cell spreading in fibrin gel, and D shows results of identifying degrees of degradation of fibrin gel by cardiac stem cells.
FIG. 19 shows results of identifying degrees of restoration of blood perfusion *in vivo* in constitutively active HIF-1α gene-introduced cardiac stem cells according to an embodiment of the present invention.
FIG. 20 shows results of identifying the ability to recover injured legs in constitutively active HIF-1α gene-introduced cardiac stem cells according to an embodiment of the present invention.
FIG. 21 shows results of identifying effects of constitutively active HIF-1α gene-introduced cardiac stem cells on angiogenesis according to an embodiment of the present invention. FIG. 21a shows CD31-positive cells, and FIG. 21b shows α-SMA-positive cells.
FIG. 22 shows results of identifying effects of constitutively active HIF-1α gene on inhibiting muscle damage and fibrosis *in vivo* according to an embodiment of the present invention.
FIG. 23 schematically illustrates a process of preparing a culture broth of cardiac stem cells according to an embodiment of the present invention.
FIG. 24 shows results of identifying an amount of VEGF contained in a culture broth of cardiac stem cells according to an embodiment of the present invention.
FIG. 25 shows results of identifying effects of a culture broth of cardiac stem cells on cellular migration using a wound healing scratch assay according to an embodiment of the present invention.
FIG. 26 shows results of identifying effects of a culture broth of cardiac stem cells on tube formation using a tube formation assay according to an embodiment of the present invention.
FIG. 27 schematically illustrates a mechanism of cardiac stem cells having enhanced angiogenic potential according to an embodiment of the present invention.

### Best Mode

Cardiac stem cells of the present invention are involved in differentiation, survival, proliferation, angiogenesis, migration of stem cells by activating an angiogenic gene such as VEGF, angiopoietins, FGF, PLGF, PDGF, SDF-1, and SCF and simultaneously activating a gene related to survival and proliferation of cells such as IGF, TGF-α, TGF-β, and NOS2, thereby efficiently promoting angiogenesis of cardiac stem cells. Therefore, the cardiac stem cells may be widely applied to all diseases whose symptoms may be ameliorated, alleviated, and treated by promoting angiogenesis.

As used herein, the term "stem cell" collectively refers to an undifferentiated cell having the ability to differentiate into various types of tissue cells, *i.e.,* stemness, in a broad sense. Such stem cells may be broadly classified into embryonic stem cells prepared using embryos, adult stem cells, gametes, and cancer stem cells. Among these, cardiac stem cells, as stem cells present in the heart, refer to multipotent stem cells capable of differentiating into all cells constituting the heart.

As used herein, the term "prevention" refers to all actions intended to inhibit or delay the onset of an angiogenesis-dependent disease by administering the composition of the present invention.

As used herein, the term "treatment" refers to all actions intended to ameliorate or beneficially change symptoms of an angiogenesis-dependent disease by administering the composition of the present invention.

As used herein, the term "individual" refers to a subject to which the composition of the present invention may be administered, without limitation.

As used herein, the term "pharmaceutical composition" may be formulated into capsules, tablets, granules, injectable formulations, ointments, powders, or drinks, and the pharmaceutical composition may be applied to a human as a target. The pharmaceutical composition of the present invention may further include a pharmaceutically acceptable carrier. For oral administration, the pharmaceutically acceptable carrier may be a binder, a lubricant, a disintegrator, an excipient, a solubilizer, a dispersant, a stabilizer, a suspending agent, a coloring agent, a flavoring agent, or the like. For injectable preparations, the pharmaceutically acceptable carrier may be a buffer, a preservative, an analgesic, a solubilizer, an isotonic agent, a stabilizer, or the like. For preparations for topical administration, the pharmaceutically acceptable carrier may be a base, an excipient, a lubricant, a preservative, or the like. The pharmaceutical composition of the present invention may be formulated into various forms in combination with the above-mentioned pharmaceutically acceptable carriers. For example, for oral administration, the pharmaceutical composition may be formulated into tablets, troches, capsules, elixirs, suspensions, syrups, wafers, and the like. For injectable preparations, the pharmaceutical composition may be formulated into a single-dose ampoule or multidose form. The pharmaceutical composition may also be formulated into sugarcoated tablets, gels, pills, powders, granules, suppositories, formulations for external use, solutions, suspensions, sustained-release preparations, slurries, and the like. Meanwhile, examples of the carrier, excipient, and diluent suitable for formulation may include lactose, dextrose, sucrose, sorbitol, mannitol, xylitol, erythritol, maltitol, starch, Acacia rubber, alginate, gelatin, calcium phosphate, calcium silicate, cellulose, methyl cellulose, amorphous cellulose, polyvinyl pyrrolidone, water, methylhydroxy benzoate, propylhydroxy benzoate, talc, magnesium stearate, or mineral oils. Also, the pharmaceutical composition may further include a filler, an anti-coagulant, a lubricant, a humectant, a flavoring agent, an emulsifier, a preservative, and the like.

A route of administration of the pharmaceutical composition of the present invention may be, but is not limited to, oral or parental administration, and may include, for example, oral, intravenous, intramuscular, intraarticular, intrabursal, intraarterial, intramedullary, intrathecal, intracardiac, transdermal, intradermal, subcutaneous, intraperitoneal, intranasal, intestinal, topical, sublingual, rectal, intrasternal, intralesional, and intracranial administration.

Dosage of the pharmaceutical composition of the present invention may vary according to various factors such as activity of a particular compound used therefor, age, body weight, health status, and gender of a patient, diet, administration time, administration route, excretion rate, combination of drugs, and severity of a disease to be prevented or treated and may be appropriately determined by those of ordinary skill in the art according to status or body weight of a patient, severity of a disease, formulation of a drug, administration route, and administration period, and a daily dosage of the composition may be 0.0001 mg/kg to 500 mg/kg or 0.001 mg/kg to 500 mg/kg. The composition may be administered in a bolus or in multiple doses. The dosage does not limit the scope of the present invention in any way.

Hereinafter, the present invention will be described in more detail with reference to the following examples. However, the following examples are merely presented to exemplify the present invention, and the scope of the present invention is not limited thereto.

### Example

### Example 1: Isolation of cardiac stem cell

All animal experiments and procedures were performed in compliance with research protocols approved by the Institutional Animal Care and Use Committee of Ewha Womans University. In order to isolate cardiac stem cells (CSCs), myocardial slices were primarily obtained from cardiac tissue of Sprague Dawley (SD) rats. Subsequently, the obtained myocardial slices were incubated for 14 days in fibrin gel prepared using 2.5 mg/mL fibrinogen and 0.5 U/mL thrombin. After the incubation, cardiac stem cells, which had migrated from the myocardial slices to the fibrin gel and grown, were isolated using urokinase, and the isolated cardiac stem cells were incubated in a cardiac stem cells (CSC) culture medium (mixture of DMEM and F-12 mixed in a volume % of 1:1 supplemented with 10% fetal bovine serum (FBS), 1% antibiotic-antifungal agent (Gibco), 10 ng/mL EGF, 2 ng/mL bFGF, and 10 ng/mL IGF-1). Then, cardiac stem cells which had been grown for 9 to 13 passages were used in all experiments. As a control, bone marrow mesenchymal stem cells (BMSCs) obtained from Inje University were used, and the bone marrow mesenchymal stem cells had been grown for 6 passages.

### Example 2: Comparison of angiogenesis-related gene expression between cardiac stem cells and bone marrow mesenchymal stem cells

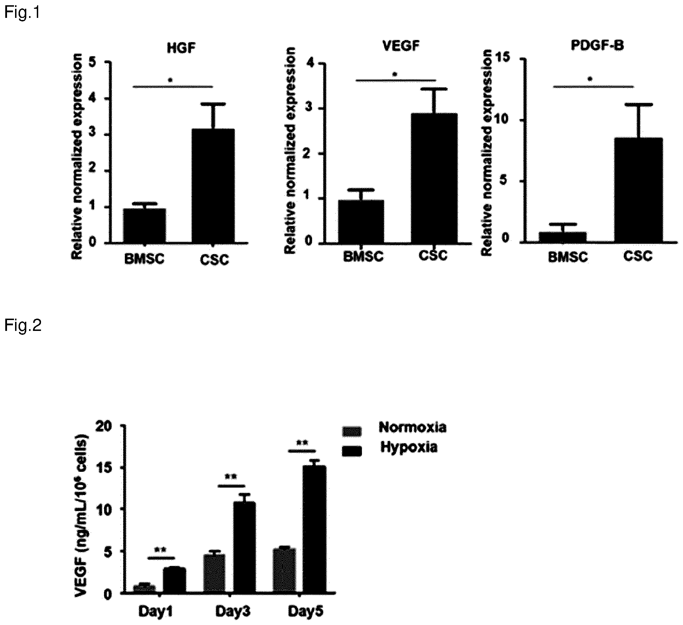

For comparison of expression levels of angiogenesis-related (proangiogenic) genes between cardiac stem cells and bone marrow mesenchymal stem cells, the stem cells were respectively inoculated onto a 6-well plate at a density of 2×10⁵ cells/well, followed by incubation for 24 hours in a CSC culture medium. Subsequently, the culture medium was replaced with a DMEM supplemented with a 1% bovine calf serum, followed by incubation for 24 hours. Then, expression levels of vascular endothelial growth factor (VEGF), platelet-derived growth factor (PDGF), and hepatocyte growth factor (HGF), which are angiogenesis-related genes, were identified by quantitative real-time PCR (qRT-PCR). More specifically, total RNA was isolated by using a TRlzol reagent, and an amount of RNA was measured using a NanoDrop2000. Then, cDNA was synthesized using an iScript^{™} cDNA synthesis kit and 1 µg of the isolated RNA as a template and amplified using a CFX96^{™} Real-Time PCR system. Primers used in the experiments are shown in Table 1. Expression levels of the genes were measured using a 2 -ΔΔCt method and β-actin as an internal control. All of the experiments were each repeated three times or more, and the results are shown as mean values ± standard deviation. Statistical significance was identified by Student's t-test for two groups and one-way ANOVA for three or more groups. A P value less than 0.05 (P < 0.05) was regarded as statistically significant, and * represents P < 0.05, ** represents P < 0.01, and *** represents P < 0.001. The results are shown in FIG. 1.

**[Table 1]**

| **Gene name** | **Sequence (5' -> 3')** | **SEQ ID NO:** |
|---|---|---|
| VEGF forward primer | GGAGTACCCCGATGAGATAGAGT | 1 |
| VEGF reverse primer | CTATGTGCTGGCTTTGGTGAG | 2 |
| PDGF-B forward primer | TGGAGTCGAGTCGGAAAGCT | 3 |
| PDGF-B reverse primer | GAAGTTGGCATTGGTGCGAT | 4 |
| bFGF forward primer | GCGACCCACACGTCAAACTA | 5 |
| bFGF reverse primer | CAGCCGTCCATCTTCCTTCA | 6 |
| Ang-1 forward primer | CTCGCTGCCATTCTGACTACAC | 7 |
| Ang-1 reverse primer | GACAGTTGCCATCGTGTTCTG | 8 |
| β-actin forward primer | AGATCAAGATCATTGCTCCTCCT | 9 |
| β-actin reverse primer | ACGCAGCTCAGTAACAGTCC | 10 |

As shown in FIG. 1, upon comparison with the bone marrow mesenchymal stem cells, it was confirmed that the expression levels of the HGF gene, the VEGF gene, and the PDGF-B gene were increased by about 3.2 times, about 2.9 times, and about 8.7 times, respectively, in the cardiac stem cells. Both the HGF and the VEGF have been known to promote angiogenesis by forming blood vessels and exhibiting cellular protective effects, and the PDGF-B is also known to have a significant role in blood vessel formation. Based thereon, it was confirmed that cardiac stem cells have higher angiogenic potential than bone marrow mesenchymal stem cells.

### Example 3: Confirmation of effect of hypoxic condition on angiogenesis of cardiac stem cell

In order to identify effects of hypoxic conditions on angiogenesis of cardiac stem cells, the cardiac stem cells were inoculated onto a 24-well plate at a density of 2×10⁴ cells/well and incubated in a CSC culture medium for 24 hours. Subsequently, the culture medium was replaced with DMEM supplemented with 1% bovine calf serum, and the cells were incubated for 24 hours, and then further incubated under normoxic conditions or hypoxic conditions (1% O₂, 5% CO₂, and 94% N₂) for 5 days. The culture medium was replaced with new one every two days, and the supernatant of each medium was collected on days 1, 3, and 5 and centrifuged at 4°C at 1,500 rpm for 10 minutes, and then an amount of the VEGF contained in the medium was measured using an ELISA kit. The results are shown in FIG. 2.

As shown in FIG. 2, it was confirmed that the amount of the VEGF significantly increased under the hypoxic conditions, and a secretion amount of the VEGF increased as the incubation time increased.

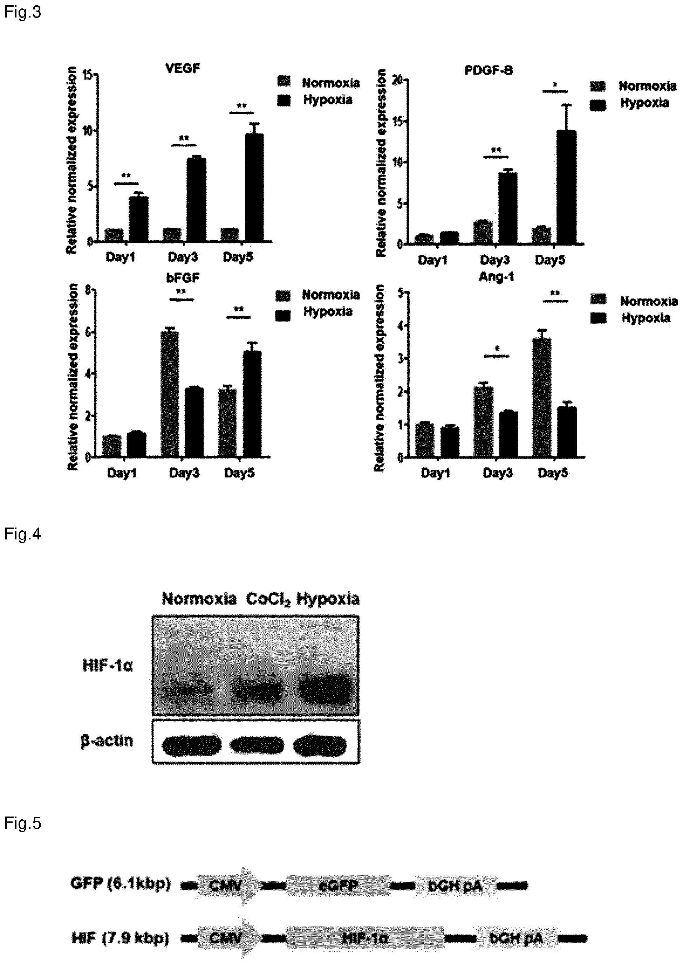

Also, expression levels of the VEGF, PDGF-B, bFGF, and Ang-1 genes, known as angiogenesis-related genes, were identified by performing qRT-PCR in the same manner as in Example 2. The results are shown in FIG. 3.

As shown in FIG. 3, the expression levels of the VEGF and the PDGF-B continuously increased under the hypoxic conditions. Although the bFGF exhibited the highest expression level on day 3 under the normoxic conditions, it was confirmed that the expression level increased over time under the hypoxic conditions. It was confirmed that the expression level of the Ang-1 gene was significantly high under the normoxic conditions.

In addition, in order to identify effects of hypoxic conditions on expression of the hypoxia-inducible factor 1-alpha (HIF-1α) of cardiac stem cells, the cardiac stem cells were inoculated onto a 6-well plate at a density of 2×10⁵ cells/well and incubated in a CSC culture medium for 24 hours. Subsequently, the culture medium was replaced with DMEM supplemented with 1% bovine calf serum, and the cells were incubated for 24 hours, and then further incubated for 5 days under normoxic conditions or hypoxic conditions (1% O₂, 5% CO₂, and 94% N ₂), or in a culture medium treated with 200 µM CoCl₂. The CoCl₂, which has a role in stabilization of the HIF-1α, was used as a positive control. After 5 days of incubation, the stem cells were lysed using a cold RIPA buffer containing a 1% protease inhibitor cocktail, and cell lysates were centrifuged at 4°C at 14,000 rpm for 10 minutes. Then, after proteins contained in the supernatant were mixed with a 2× laemmli sample buffer, the mixture was heated at 95°C for 5 minutes to prepare a protein sample. The prepared protein sample was electrophoresed using 10% (w/v) SDS-PAGE and then analyzed using a common western blotting method. HIF-1α antibody (Novus Biologicals) and β-actin antibody (Novus Biologicals) were used as primary antibodies, and anti-mouse peroxidase-conjugated secondary antibody (Millipore) was used as a secondary antibody. The results are shown in FIG. 4.

As shown in FIG. 4, the expression level of the HIF-1α was increased by about 1.7 times under the CoCl₂-treated conditions and increased by about 3.0 times or more under the hypoxic conditions.

Based on the above-described results, it was confirmed that the expression of angiogenesis-related genes such as the HIF-1α, VEGF, PDGF-B, and bFGF genes significantly increased in the cardiac stem cells under the hypoxic conditions.

### Example 4: Confirmation of effect of HIF-1α gene on cardiac stem cell

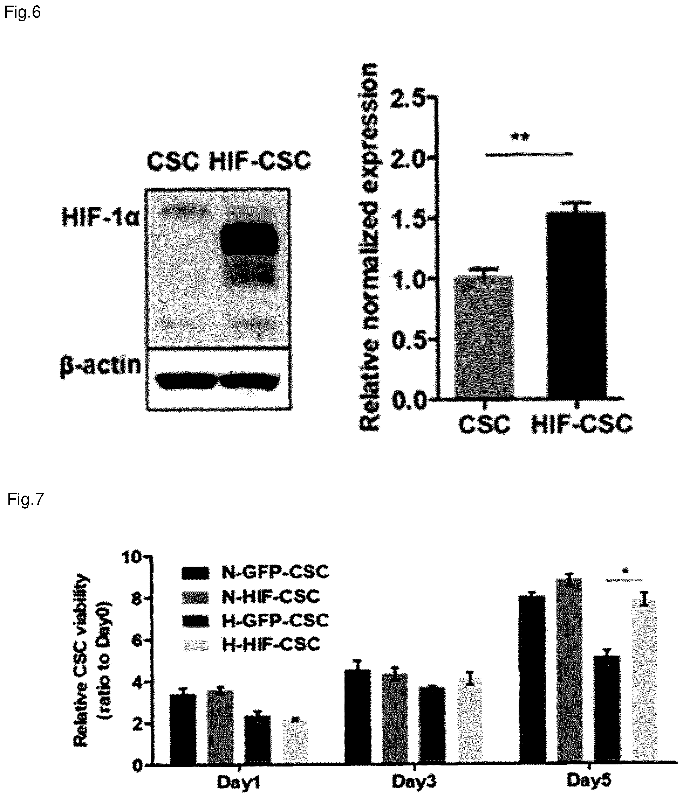

In order to identify effects of the HIF-1α gene on cardiac stem cells, vectors constructed to express eGFP or HIF-1α gene (GenBank: NM_024359.1, SEQ ID NO: 11) were purchased from DetaiBio Co., Ltd. (FIG. 5). Cardiac stem cells were transfected with the constructed vectors using a Neon transfection system. In order to confirm whether the HIF-1α gene is overexpressed in the transfected cardiac stem cells, western blotting was performed in the same manner as in Example 3. The results of the western blotting were quantified by using ImageJ software. The results are shown in FIG. 6.

As shown in FIG. 6, it was confirmed that the HIF-1α gene was overexpressed in the transfected cardiac stem cells. Based on the results, it was confirmed that the cardiac stem cells were properly transfected with the HIF-1α vector.

Subsequently, in order to confirm the viability of the transfected cardiac stem cells, the transfected cardiac stem cells were inoculated onto a 96-well plate at a density of 5×10³ cells/well and incubated in a CSC culture medium for 24 hours. Then, the culture medium was replaced with DMEM supplemented with 1% bovine calf serum, 50 µg/mL ascorbic acid, and 0.1 µM) dexamethasone, and the cells were incubated under the normoxic conditions (N) or hypoxic conditions (H). Then, the viability was identified using a CCK-8 assay. The results are shown in FIG. 7.

As shown in FIG. 7, it was confirmed that the viability of the cardiac stem cells overexpressing the eGFP gene rapidly decreased under the hypoxic conditions, while the viability of the cardiac stem cells overexpressing the eGFP gene or overexpressing the HIF-1α gene was not affected under the normoxic conditions. However, it was confirmed that the viability of the cardiac stem cells overexpressing the HIF-1α gene was maintained under the hypoxic conditions like the normoxic conditions. Based on the results, it was confirmed that the overexpression of the HIF-1α gene enhanced the cellular viability by inhibiting cease of growth, apoptosis, or the like induced by the hypoxic conditions.

### Example 5: Confirmation of therapeutic effect of HIF-1α gene-introduced cardiac stem cell in vitro

### 5.1. Wound healing scratch assay

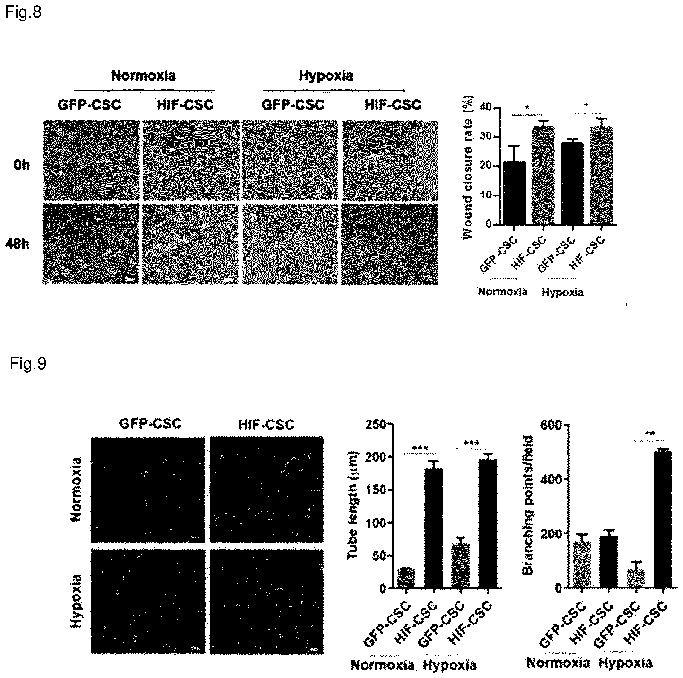

In order to identify effects of HIF-1α gene-introduced cardiac stem cells on healing a wound, cardiac stem cells transfected in the same manner as in Example 4 were inoculated onto a 6-well plate at a density of 3×10⁵ cells/well and incubated in a CSC culture medium for 24 hours. Subsequently, after the culture medium was replaced with DMEM supplemented with 1% bovine calf serum, the cells were further incubated for 24 hours and then removed therefrom using a 200 µL tip. Then, the cells were washed three times with phosphate buffered saline (PBS) to remove all detached cells, and the remaining cells were incubated for 48 hours. Thereafter, the cells were observed using an optical microscope, and a space re-filled with cells was measured using ImageJ software. Subsequently, the cells were quantified by substituting areas of the space into the following formula "Wound closure rate (%) = 100 × (A₀ - A₂₄)/A₀". In the formula, A₀ represents an area of an empty space not containing cells at time 0, and A₂₄ represents an area of an empty space not containing cells after 24 hours. The results are shown in FIG. 8.

As shown in FIG. 8, it was confirmed that the cardiac stem cells introduced with the HIF-1α gene had a higher wound closure rate compared to the cardiac stem cells introduced with the eGFP gene. Based on the results, it was confirmed that the overexpression of the HIF-1α gene promoted migration of the cardiac stem cells.

### 5.2. Tube formation assay

In order to identify effects of HIF-1α gene-introduced cardiac stem cells on angiogenesis, an experiment was performed using an *In Vitro* Angiogenesis assay kit (Trevigen, Inc.). More specifically, 50 µL of a Basement Membrane Extract (BME) solution was added to each well of a 96-well plate, followed by gelation at 37°C for 30 minutes. Subsequently, cardiac stem cells transfected in the same manner as in Example 4 were stained with a 2 µM calcein-AM solution, inoculated onto the BMEcoated wells at a density of 1×10⁴ cells/well, and incubated for 5 hours. Degrees of tube formation were observed using a fluorescence microscope, and images obtained therefrom were analyzed using ImageJ software. The results are shown in FIG. 9.

As shown in FIG. 9, although tubes were formed in the case of cardiac stem cells introduced with the eGFP both under the normoxic conditions and the hypoxic conditions, it was confirmed that tubes were formed in a scattered form throughout a matrix under the hypoxic conditions. In contrast, a tube formation rate was increased in the case of cardiac stem cells introduced with the HIF-1α, and it was confirmed that the cardiac stem cells formed a normal tubular structure rather than a scattered form, particularly under the hypoxic conditions. Based on the results, it was confirmed that overexpression of the HIF-1α promoted tube formation under the hypoxic conditions.

### 5.3. Confirmation of effect of HIF-1α gene on differentiation efficiency of vascular endothelial cell

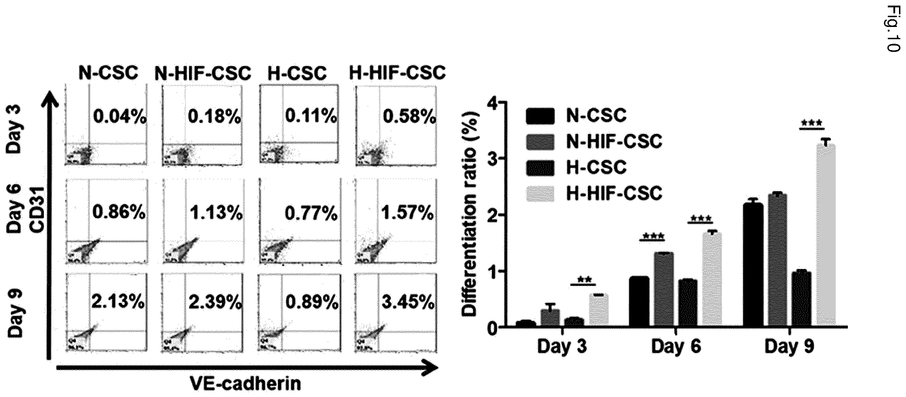

In order to identify effects of HIF-1α gene-introduced cardiac stem cells on differentiation efficiency of vascular endothelial cells, cardiac stem cells or HIF-1α gene-introduced cardiac stem cells were incubated in an EGM-2 culture medium supplemented with 50 ng/mL VEGF and 10 ng/mL bFGF. The culture medium was replaced with a new one every three days, and cardiac stem cells were collected on days 3, 6, and 9 and analyzed using a vascular endothelial cell marker. More specifically, 1×10⁶ cells were added to a FACS buffer (10% FBS in PBS) and reacted using APC-conjugated CD31 antibody (Miltenyi Biotec) or FITC-conjugated VEcadherin antibody (Novus) for 1 hour, and then all unbound antibodies were removed by washing twice using PBS. Then, fluorescence was measured using a FACSCalibur cell analyzer, and degrees of fluorescence were quantified using FlowJo software. The results are shown in FIG. 10.

As shown in FIG. 10, it was confirmed that the HIF-1α gene-introduced cardiac stem cells had a significantly higher VE-cadherin-positive/CD31-positive cell ratio than the cardiac stem cells under the hypoxic conditions. Based on the results, it was confirmed that overexpression of the HIF-1α gene under the hypoxic conditions promoted differentiation into vascular endothelial cells.

### Example 6: Confirmation of therapeutic effect of HIF-1α gene-introduced cardiac stem cell in vivo

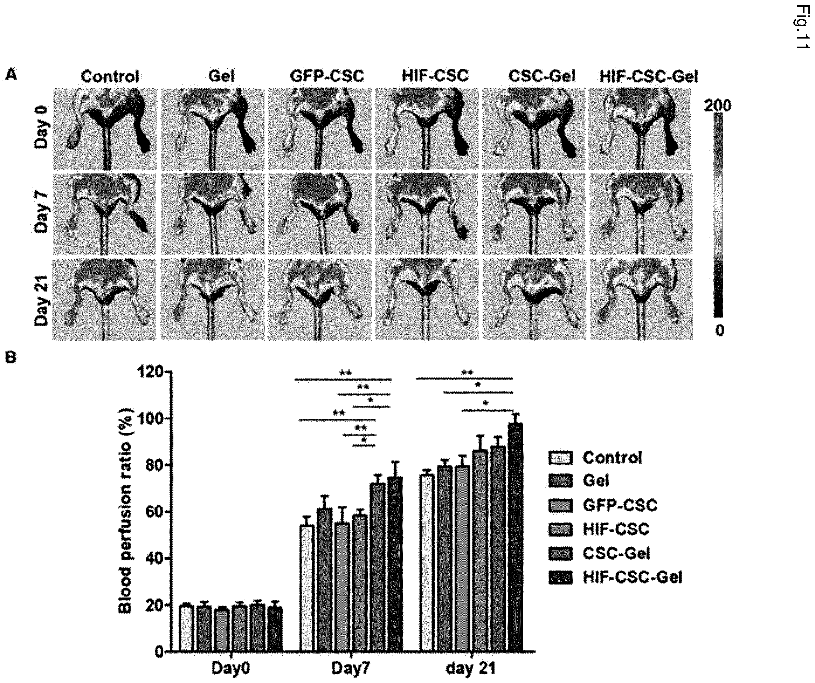

In order to construct an ischemia/reperfusion injury (hind limb ischemia; HLI) animal model, first, 9-week-old BALB/c female mice were anesthetized using 1% pentobarbital sodium at a dose of 90 mg/kg. The HLI animal model was constructed by isolating femoral artery from blood vessels and nerves, followed by ligation and removal from proximal origins of branches of the femoral artery to distal branches of the saphenous artery. Then, after one day, 2×10⁶ cardiac stem cells introduced with eGFP or HIF-1α were mixed with 50 µL of PBS or 50 µL of fibrin gel and injected intramuscularly. The injections were administered to two sites of gastrocnemius muscle and one site of adductor muscle in the injured leg. Blood perfusion was observed using a laser Doppler perfusion imager on day 0, when the animal model was constructed, and on day 7 and day 21 after the cardiac stem cells were injected. Control represents a control injected with the same amount of PBS, and Gel represents a control injected with the same amount of fibrin gel. The results are shown in FIG. 11.

As shown in FIG. 11, based on the results observed on day 21, it was confirmed that the blood perfusion ratio increased in the case of injecting the HIF-1α gene-introduced cardiac stem cells (HIF-CSC) compared to the eGFP gene-introduced cardiac stem cells (GFP-CSC), and the blood perfusion ratio of the case of injecting the HIF-1α gene-introduced cardiac stem cells (HIF-CSC-Gel) together with the fibrin gel was 97.57% ± 4.29%, which is similar to that of normal mice.

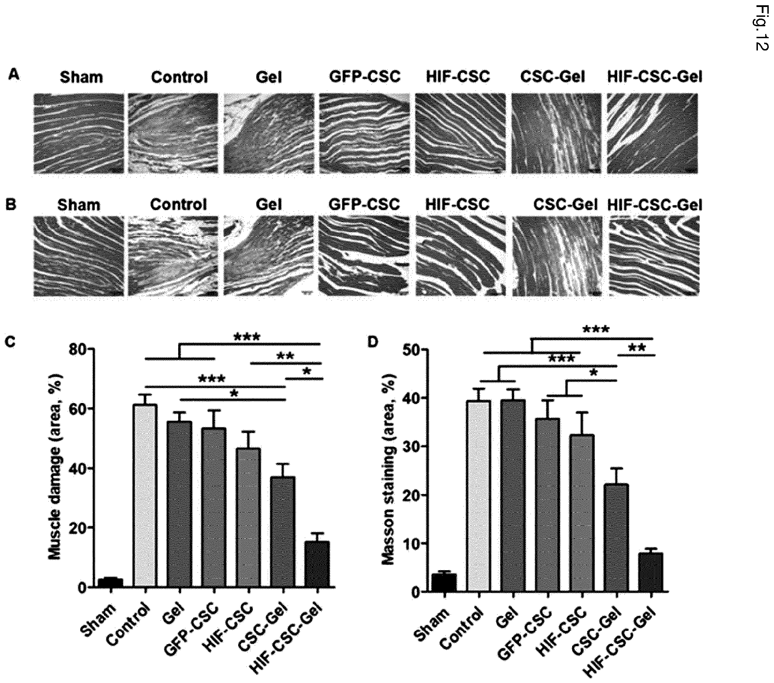

In addition, in order to identify the degree of recovery of muscle tissue, the mice were euthanized on day 21 after the cardiac stem cells were injected, muscle tissue was obtained therefrom, and histological changes were identified. The degree of degradation of muscle tissue was observed by Hematoxylin-eosin (H&E) staining (Abcam), and the degree of fibrosis was observed by Masson's trichrome (MT) staining (Polysciences). Then, these results were quantified using ImageJ software. The results are shown in FIG. 12.

As shown in FIG. 12, it was confirmed that degradation of muscles and inflammatory responses increased in the controls injected with PBS (Control) or fibrin gel (Gel), and these results were caused because nutrients and oxygen were not supplied due to angiostenosis. In the case of the cardiac stem cells injected using the PBS, in comparison with the eGFP gene-introduced cardiac stem cells (GFP-CSC), the degree of damage and the degree of fibrosis decreased in the muscles by the HIF-1α gene-introduced cardiac stem cells (HIF-CSC), and it was confirmed that the degrees of damage and fibrosis were similar to those of normal mice in the case of using the fibrin gel (HIF-CSC-Gel).

Based on the results, it was confirmed that the overexpression of the HIF-1α gene effectively recovered injured blood vessels, thereby efficiently inhibiting damage to muscle, fibrosis, inflammatory response, and the like.

### Example 7: Constitutively active HIF-1α gene-introduced cardiac stem cell

### 7.1. Construction of constitutively active HIF-1α gene-introduced cardiac stem cell

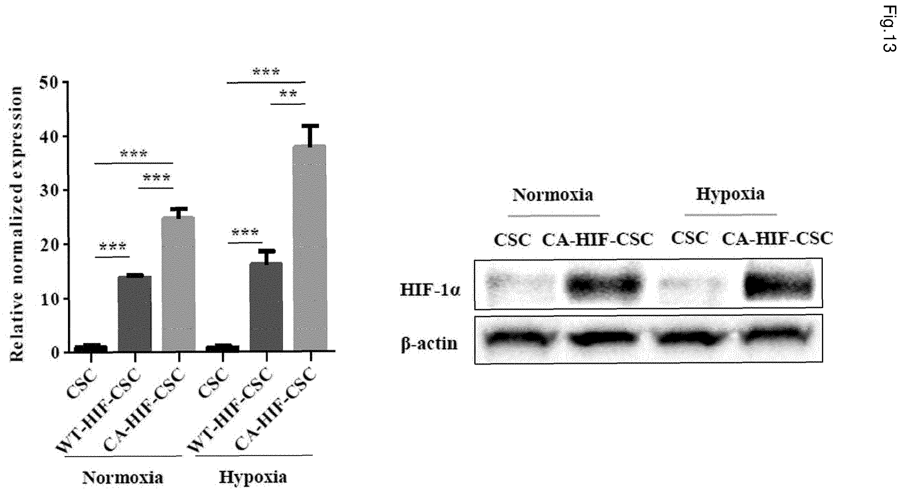

In order to construct cardiac stem cells in which a constitutively active HIF-1α gene is expressed, *i.e.,* the HIF-1α gene is stably expressed regardless of environmental conditions, a vector, which was constructed to express a mutant (SEQ ID NO: 12) having a deleted amino acid sequence of 392^{nd} to 520^{th} amino acids from the HIF-1α protein and two missense mutations (Pro567Thr and Pro658Gln), was purchased from DetaiBio Co., Ltd. Cardiac stem cells were transfected with the constructed vector using a Neon transfection system. In order to identify whether the transfected cardiac stem cells overexpress the HIF-1α gene, western blotting was performed in the same manner as in Example 3. The results of the western blotting were quantified by using ImageJ software. The results are shown in FIG. 13.

As shown in FIG. 13, it was confirmed that the constitutively active HIF-1α was overexpressed in the transfected cardiac stem cells, and the expression level of the mutant HIF-1α gene (CA-HIF-CSC) increased compared to the wild-type HIF-1α gene. Based on the results, it was confirmed that the constitutively active HIF-1α vector was properly transfected.

### 7.2. Confirmation of expression level of angiogenesis-related gene in constitutively active HIF-1α gene-introduced cardiac stem cell

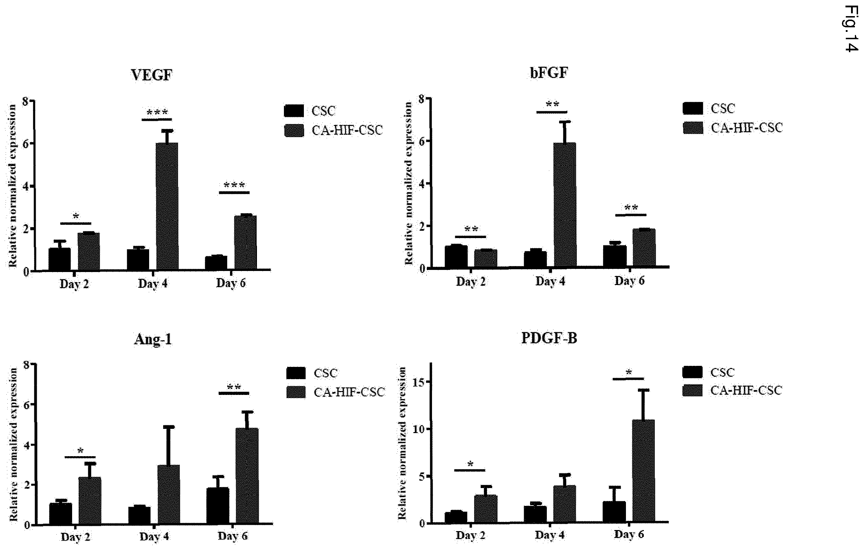

In order to identify expression levels of angiogenesis-related genes in constitutively active HIF-1α gene-introduced cardiac stem cells, qRT-PCR was performed in the same manner as in Example 2. The results are shown in FIG. 14.

As shown in FIG. 14, it was confirmed that expression of all of the VEGF, bFGF, Ang-1, and PDGF-B genes increased in comparison with the cardiac stem cells. Based on the results, it was confirmed that the constitutively active HIF-1α gene-introduced cardiac stem cells had increased angiogenic potential.

### 7.3. Wound healing scratch assay

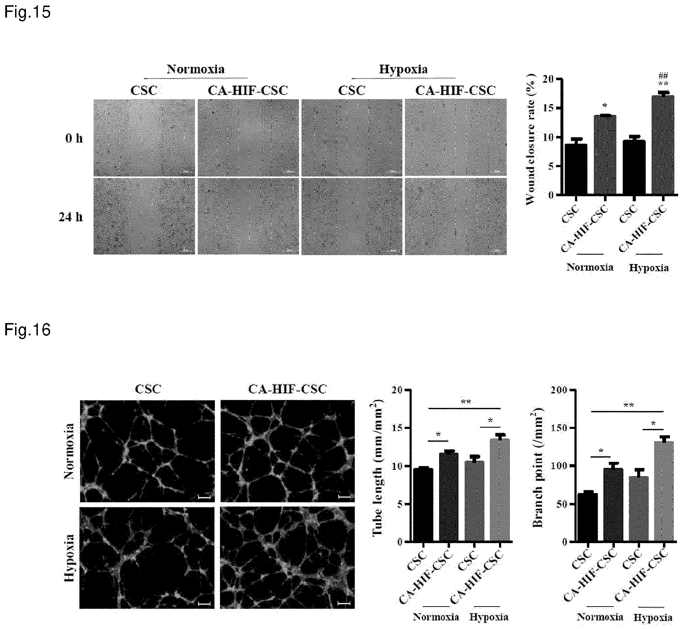

In order to identify effects of the constitutively active HIF-1α gene-introduced cardiac stem cells on wound healing, a wound healing scratch assay was conducted in the same manner as in Example 5.1. The results are shown in FIG. 15.

As shown in FIG. 15, it was confirmed that the constitutively active HIF-1α gene-introduced cardiac stem cells had a higher wound closure rate compared with the cardiac stem cells. Based on the results, it was confirmed that overexpression of the constitutively active HIF-1α gene promoted migration of the cardiac stem cells.

### 7.4. Tube formation assay

In order to identify effects of the constitutively active HIF-1α gene-introduced cardiac stem cells on angiogenesis, a tube formation assay was conducted in the same manner as in Example 5.2. The results are shown in FIG. 16.

As shown in FIG. 16, it was confirmed that tube formation was increased in the constitutively active HIF-1α gene-introduced cardiac stem cells both under the normoxic conditions and the hypoxic conditions, and the tube had a normal tubular structure in comparison with the cardiac stem cells. Based on the results, it was confirmed that overexpression of the constitutively active HIF-1α gene promoted formation of a tubular structure.

### 7.5. Confirmation of effect of constitutively active HIF-1α gene on differentiation efficiency of vascular endothelial cell

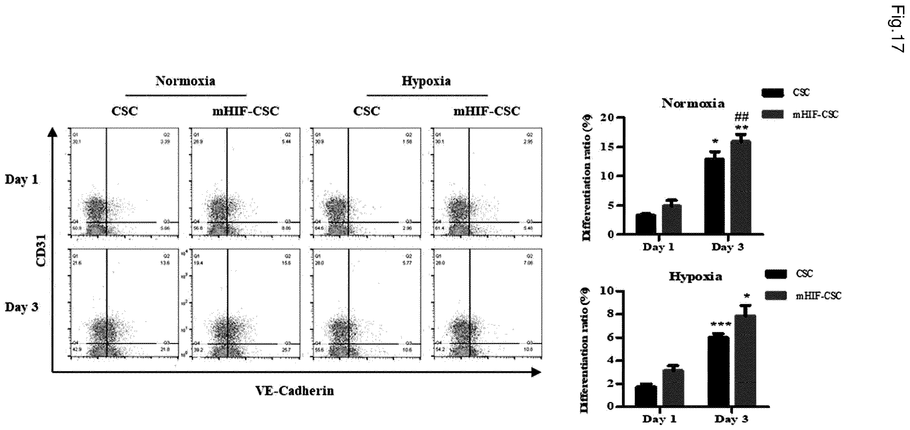

In order to confirm effects of constitutively active HIF-1α gene-introduced cardiac stem cells on differentiation efficiency of vascular endothelial cells, VEcadherin-positive/CD31-positive cell ratios were measured in the same manner as in Example 5.3. The results are shown in FIG. 17.

As shown in FIG. 17, it was confirmed that the constitutively active HIF-1α gene-introduced cardiac stem cells had a significantly higher VE-cadherinpositive/CD31-positive ratio than general cardiac stem cells. Based on the results, it was confirmed that overexpression of the constitutively active HIF-1α gene promoted differentiation into vascular endothelial cells.

### 7.6. Confirmation of characteristics of constitutively active HIF-1α gene-introduced cardiac stem cell encapsulated in fibrin gel

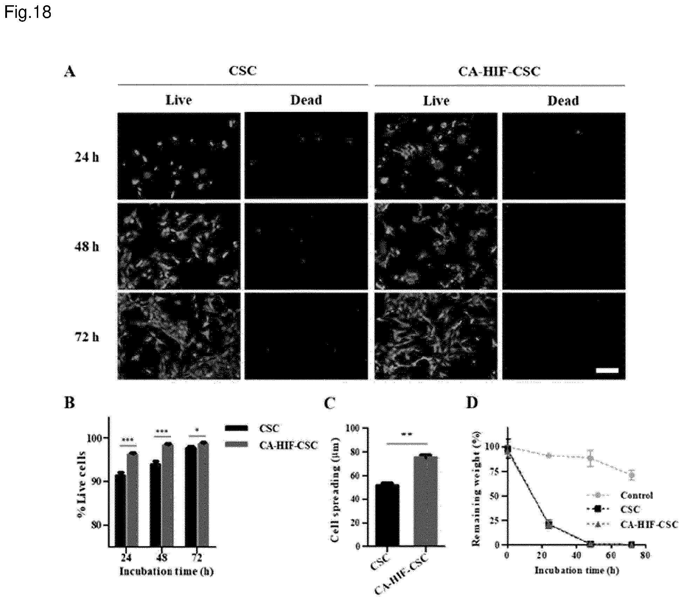

In order to identify characteristics of constitutively active HIF-1α gene-introduced cardiac stem cells encapsulated in fibrin gel, first, survival of the cardiac stem cells contained in fibrin gel was examined using a Live/Dead^{®} viability/cytotoxicity assay kit (Invitrogen). The results are shown in FIGS. 18A and 18B.

As shown in FIGS. 18A and 18B, it was confirmed that the constitutively active HIF-1α gene-introduced cardiac stem cells had a significantly higher viability than general cardiac stem cells.

Also, the degree of cell spreading in fibrin gel was identified using a fluorescent image. The results are shown in FIG. 18C.

As shown in FIG. 18C, it was confirmed that more constitutively active HIF-1α gene-introduced cardiac stem cells spread in fibrin gel.

In order to identify whether the cardiac stem cells encapsulated in fibrin gel have the ability to degrade the fibrin gel, 5×10⁴ cells/mL cardiac stem cells were mixed with fibrin gel prepared by mixing a 2.5 mg/mL fibrinogen solution and 0.5 U/mL thrombin. After incubation, the culture medium was removed at 0 hours, 24 hours, 48 hours, and 72 hours, followed by washing three times using PBS. After the remaining fibrin gel was lyophilized, the degree of degradation of the fibrin gel was evaluated in comparison with dry weight of the fibrin gel at 0 hours of incubation without the cells. The results are shown in FIG. 18D.

As shown in FIG. 18D, it was confirmed that both the general cardiac stem cells and the constitutively active HIF-1α gene-introduced cardiac stem cells degraded the fibrin gel.

### 7.7. Confirmation of effect of constitutively active HIF-1α gene-introduced cardiac stem cells on restoration of perfusion in vivo

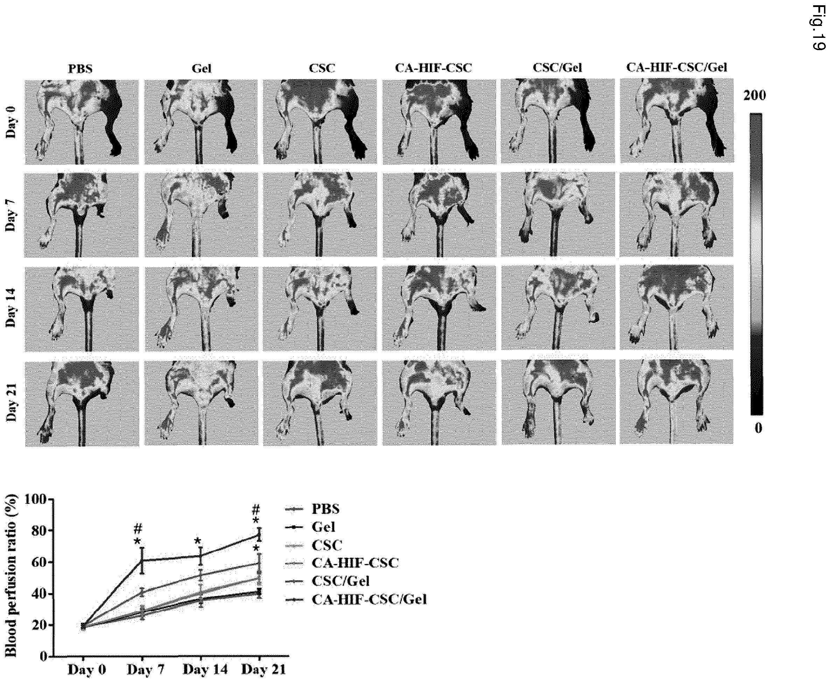

In order to identify whether constitutively active HIF-1α gene-introduced cardiac stem cells restore perfusion by healing injured blood vessels *in vivo,* the degree of reperfusion was identified in an ischemia/reperfusion injury animal model in the same manner as in Example 6. The results are shown in FIG. 19.

As shown in FIG. 19, it was confirmed that the blood perfusion was restored to a degree similar to a normal level in the case of injecting the constitutively active HIF-1α gene-introduced cardiac stem cells encapsulated in fibrin gel (CA-HIF-CSC/Gel).

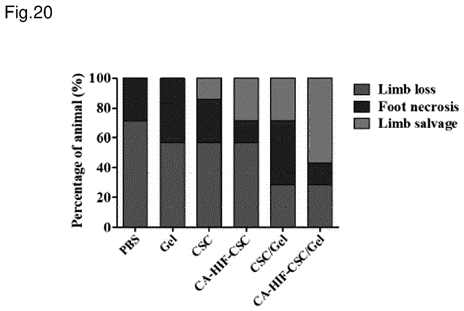

Also, degrees of limb loss, foot necrosis, and limb salvage of damaged legs were measured using the number of mice and expressed as percentages. The results are shown in FIG. 20.

As shown in FIG. 20, it was confirmed that the recovery rate was the highest in the case of injecting the constitutively active HIF-1α gene-introduced cardiac stem cells encapsulated in fibrin gel.

### 7.8. Confirmation of effect of constitutively active HIF-1α gene-introduced cardiac stem cell on angiogenesis in vivo

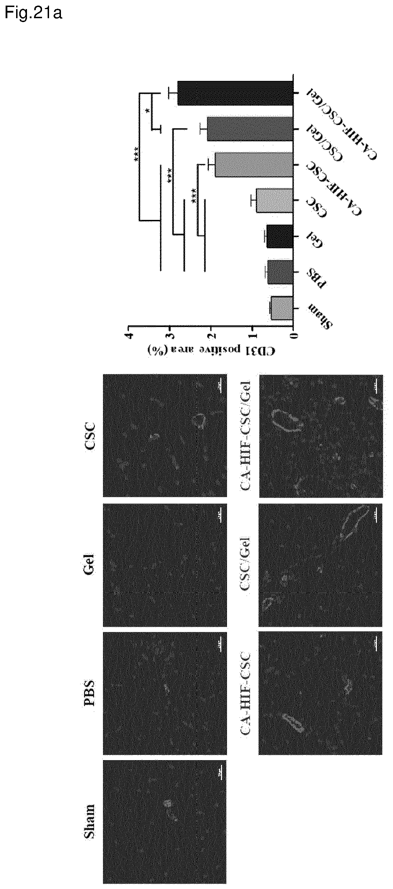

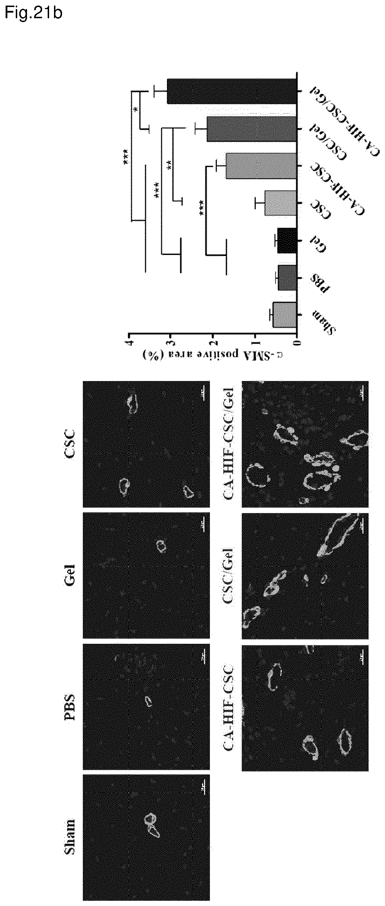

In order to identify whether constitutively active HIF-1α gene-introduced cardiac stem cells form new blood vessels *in vivo,* cardiac stem cells were transplanted into an ischemia/reperfusion injury animal model in the same manner as in Example 6, and CD31-positive and α-SMA-positive cells were identified in the injured leg tissue after 21 days. The results are shown in FIGS. 21a and 21b.

As shown in FIGS. 21a and 21b, it was confirmed that the numbers of the CD31-positive and α-SMA-positive cells significantly increased in the case of injecting the constitutively active HIF-1α gene-introduced cardiac stem cells encapsulated in fibrin gel.

### 7.9. Confirmation of therapeutic effect of constitutively active HIF-1α gene-introduced cardiac stem cell in vivo

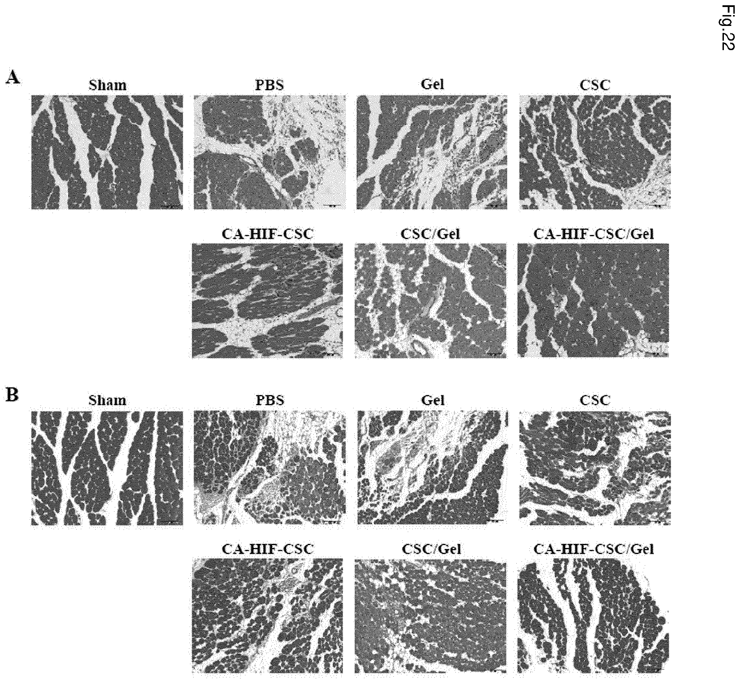

In order to identify therapeutic effects of constitutively active HIF-1α gene-introduced cardiac stem cells *in vivo,* H&E staining and MT staining were conducted in the same manner as in Example 6. The results are shown in FIG. 22.

As shown in FIG. 22, it was confirmed that tissue similar to that of the normal control (Sham) was obtained in the case of injecting the constitutively active HIF-1α gene-introduced cardiac stem cells encapsulated in fibrin gel.

Based on the results, it was confirmed that the constitutively active HIF-1α gene-introduced cardiac stem cells according to the present invention may not only normalize blood flow by restoring injured blood vessels but also effectively inhibit inflammatory response, necrosis, and the like.

### Example 8: Confirmation of paracrine effect of cardiac stem cell

### 8.1. Preparation of culture broth of cardiac stem cell

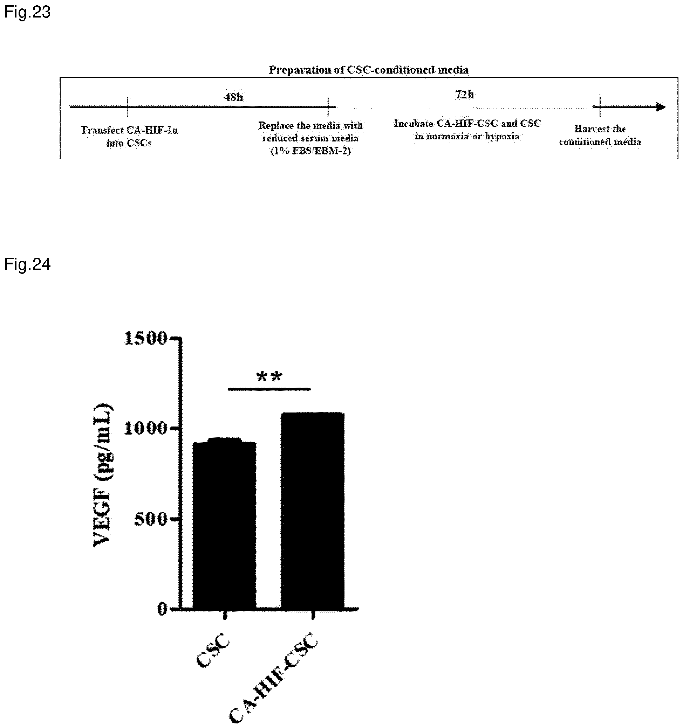

In order to prepare a culture broth of cardiac stem cells, constitutively active HIF-1α gene-introduced cardiac stem cells constructed in the same manner as in Example 7.1 or general cardiac stem cells were incubated in a CSC culture medium for 48 hours, and the culture medium was replaced with EBM-2 supplemented with 1% FBS, followed by incubation for 72 hours under normoxic conditions or hypoxic conditions. Then, centrifugation was performed to obtain a supernatant not containing cells. A process of preparing the culture broth of cardiac stem cells is briefly shown in FIG. 23.

### 8.2. Measurement of VEGF in culture broth of cardiac stem cell

In order to measure an amount of VEGF contained the culture broth of cardiac stem cells, ELISA was performed in the same manner as in Example 3. The results are shown in FIG. 24.

As shown in FIG. 24, it was confirmed that the amount of VEGF contained in the culture broth, which was obtained after incubating the constitutively active HIF-1α gene-introduced cardiac stem cells under the normoxic conditions, was the largest. Thereafter, the culture broth of the constitutively active HIF-1α gene-introduced cardiac stem cells obtained after incubation under the normoxic conditions was used as the culture broth.

### 8.3. Wound healing scratch assay

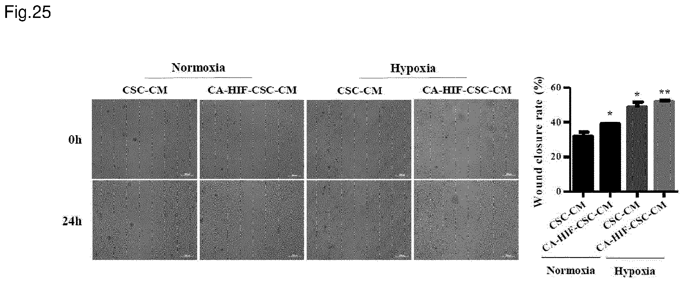

In order to identify effects of the culture broth of the HIF-1α gene-introduced cardiac stem cells on migration of vascular endothelial cells (human umbilical vein endothelial cells, HUVECs), a wound healing scratch assay was conducted in the same manner as in Example 5.1. More specifically, vascular endothelial cells were incubated instead of the cardiac stem cells and treated with mitomycin C to inhibit proliferation of the cells. Then, after removing parts of the cells using a 200 µL tip, the culture broth of cardiac stem cells was added thereto, and the degree of filling of the removed parts of the cells was observed while incubating the cells. The results are shown in FIG. 25.

As shown in FIG. 25, it was confirmed that migration of the vascular endothelial cells increased under the hypoxic conditions, and in particular, the wound closure rate was high in the case of treating with the culture broth of the constitutively active HIF-1α gene-introduced cardiac stem cells.

### 8.4. Tube formation assay

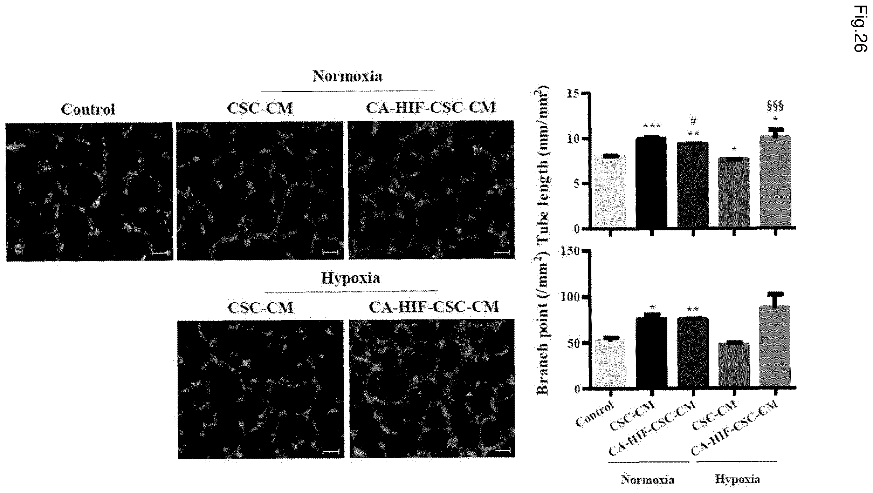

In order to identify effects of a culture broth of constitutively active HIF-1α gene-introduced cardiac stem cells on angiogenesis, a tube formation assay was conducted in the same manner as in Example 5.2. More specifically, vascular endothelial cells, instead of the cardiac stem cells, were incubated in Matrigel and treated with a culture broth of cardiac stem cells, and then the degree of tube formation was measured. The results are shown in FIG. 26.

As shown in FIG. 26, although there was no significant difference between the culture broth of cardiac stem cells and the culture broth of constitutively active HIF-1α gene-introduced cardiac stem cells under the normoxic conditions, it was confirmed that tube formation was promoted by the culture broth of constitutively active HIF-1α gene-introduced cardiac stem cells under the hypoxic conditions while tube formation was not induced by the culture broth of cardiac stem cells.

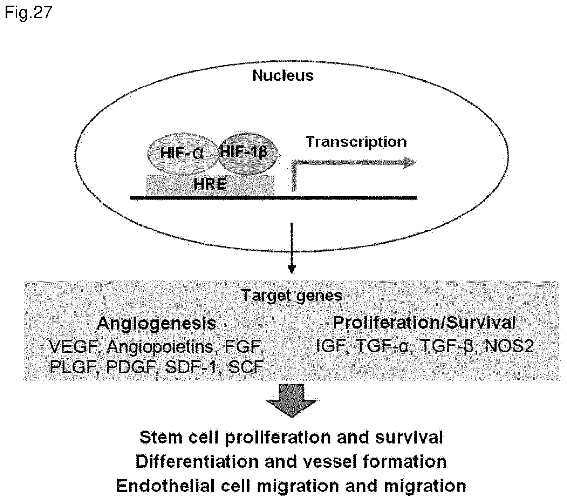

Based on the results, the cardiac stem cells, the cardiac stem cells introduced with the HIF-1α gene, *i.e.,* overexpressing the HIF-1α gene, or the culture broth thereof according to the present invention may be involved in differentiation, survival, proliferation, angiogenesis, and migration of stem cells by activating an angiogenic gene such as VEGF, angiopoietins, FGF, PLGF, PDGF, SDF-1, and SCF and simultaneously activating a gene related to survival and proliferation of cells such as IGF, TGF-α, TGF-β, and NOS2, thereby promoting angiogenesis of cardiac stem cells. A mechanism thereof is briefly shown in FIG. 27. Based thereon, it was confirmed that not only was angiogenic potential promoted, but also, injured blood vessels were efficiently recovered in the gene-introduced cardiac stem cells by introducing the HIF-1α gene or the constitutively active gene thereof into cardiac stem cells. In addition, it is expected that angiogenic potential of cardiac stem cells may be enhanced by introducing the genes whose expression levels are increased by the HIF-1α gene, such as VEGF, FGF, PDGF, and HGF, as well as the HIF-1α gene, into the cardiac stem cells, similar to the case of the HIF-1α gene.

The above description of the present invention is provided for the purpose of illustration, and it would be understood by those skilled in the art that various changes and modifications may be made without changing technical conception and essential features of the present invention. Thus, it is clear that the above-described embodiments are illustrative in all aspects and do not limit the present invention.

### [Industrial Applicability]

Because cardiac stem cells having enhanced angiogenic potential or a culture broth thereof according to the present invention may not only promote the angiogenic potential but also efficiently restore damaged blood vessels, they are expected to be efficiently used for various diseases, which may be treated by promoting angiogenesis, such as wounds, chronic ulcer, ischemic stroke, myocardial infarction, angina pectoris, and cerebrovascular dementia.

## Claims

1. A pharmaceutical composition for preventing or treating an angiogenesis-dependent disease comprising, as an active ingredient, cardiac stem cells or a culture broth thereof.

2. The pharmaceutical composition according to claim 1, wherein the cardiac stem cells are transformed with a gene encoding at least one growth factor selected from the group consisting of hypoxia-inducible factor 1-alpha (HIF-1α), vascular endothelial growth factor (VEGF), platelet-derived growth factor (PDGF), hepatocyte growth factor (HGF), and fibroblast growth factor (FGF).

3. The pharmaceutical composition according to claim 2, wherein the hypoxia-inducible factor 1-alpha comprises amino acids represented by a sequence of SEQ ID NO: 12.

4. The pharmaceutical composition according to claim 1, wherein the angiogenesis-dependent disease comprises at least one disease selected from the group consisting of wounds, chronic ulcer, ischemic stroke, myocardial infarction, angina pectoris, peripheral artery disease, critical limb ischemia, diabetic foot ulcer, and cerebrovascular dementia.

5. The pharmaceutical composition according to claim 1, further comprising hydrogel.

6. The pharmaceutical composition according to claim 5, wherein the hydrogel comprises at least one selected from the group consisting of collagen, gelatin, chondroitin, hyaluronic acid, alginic acid, Matrigel^{™}, chitosan, peptide, fibrin, polyglycolic acid (PGA), polylactic acid (PLA), polyethylene glycol (PEG), and polyacrylamide.

7. A method for preventing or treating an angiogenesis-dependent disease, the method comprising administering a composition comprising, as an active ingredient, cardiac stem cells or a culture broth thereof to an individual.

8. A use of a composition comprising, as an active ingredient, cardiac stem cells or a culture broth thereof for preventing or treating an angiogenesis-dependent disease.
